# EUROPÄISCHE PATENTSCHRIFT

(11) **EP 2 063 374 B1**
(45) Veröffentlichungstag und Bekanntmachung des Hinweises auf die Patenterteilung: **05.03.2014**
(21) Anmeldenummer: 08019805.4
(22) Anmeldetag: 13.11.2008
(51) Int. Cl.: G06F 19/00

(54) **Verfahren und Vorrichtung zur Dokumentation medizinischer Daten**
Device and method for documenting medical data
Dispositif et procédé de documentation de données médicales

(30) Priorität: 13.11.2007 EP 07022002
(43) Veröffentlichungstag der Anmeldung: 27.05.2009
(73) Patentinhaber: How to Organize (H2O) GmbH, 10115 Berlin (DE)
(72) Erfinder: Abri, Omid, Prof. Dr., 14129 Berlin (DE)
(74) Vertreter: Fugmann, Winfried

(56) Entgegenhaltungen:
- WO-A-00/57336
- WO-A-2007/073420
- DE-A1- 19 904 090
- FR-A1- 2 856 222
- US-A- 5 654 750
- US-A1- 2003 108 327
- US-A1- 2003 159 141

## Beschreibung

Die vorliegende Erfindung betrifft ein Verfahren sowie eine Vorrichtung zur Dokumentation medizinischer Daten.

Derartige Verfahren und Vorrichtungen sind bekannt. So sind beispielsweise endoskopische Bilddokumentationssysteme bekannt, mit denen bestimmte Bilddaten zusammen mit entsprechenden Patienteninformationen aufgenommen, bearbeitet und gespeichert werden können. Ein solches System wird beispielsweise unter dem Namen KARL STORZ AIDA® in verschiedenen Ausführungen angeboten.

Ein solches Bilddokumentationssystem kann über eine Schnittstelle mit einer zentralen busgestützten OP-Steuerung verbunden sein. Eine solche OP-Steuerung und ein solches Bussystem sind beispielsweise in EP 1 034 480 B1 und in WO 02/19957 A2 beschrieben. Es ist auch bekannt, über einen Datenlogger intraoperative Geräte- bzw. Funktionsparameter auf einer zentralen Steuereinheit aufzuzeichnen.

Weiterhin sind aus WO 2007/073420 A1, DE 10 2005 025 903 A1 und aus US 7,231,135 B2 Systeme zur Speicherung und Bearbeitung medizinischer Daten, insbesondere medizinischer Videodaten bekannt.

Würde bei den bekannten Systemen zur Dokumentation medizinischer Daten eine fortlaufende Aufzeichnung, insbesondere eine fortlaufende Videoaufzeichnung aller Aktivitäten im Operationssaal (OP) erfolgen, so würden nach relativ kurzer Zeit die Speichermedien überlaufen und keine weitere Speicherung mehr erfolgen können. Andererseits wäre es für eine retrospektive Qualitätskontrolle im OP wünschenswert, auf eine vollständige Videoaufzeichnung zurückgreifen zu können.

In der gattungsfremden Offenlegungsschrift US 2003/0108327 A1 wird ein Bildaufzeichnungssystem beschrieben, bei dem Bilddaten in einem zyklischen Speicher gespeichert und regelmäßig wieder überschrieben werden. Nach Generierung eines Alarmsignals erfolgt eine weitere Speicherung von Bilddaten mit unveränderter Bildfrequenz, jedoch verbleiben zuvor gespeicherte Bilddaten mit verringerter Bildfrequenz auf dem Speichermedium.

Die Patentschrift US 5,654,750 beschreibt ein automatisches Aufnahme- und Speichersystem für ein Krankenhaus. Die Videodaten werden nach außerhalb des Krankenhauses übertragen und auf Speichermedien, insbesondere Videorekordern, gespeichert. Das System kann mehrere Videorekorder umfassen für eine ununterbrochene, überlappende Speicherung aller Aufnahmen, wenn der regelmäßig notwendige Wechsel der Videobänder erfolgt. Um Speicherplatz zu sparen, werden die Videorekorder automatisch gestartet bzw. wieder angehalten, wenn über im OP angeordnete Bewegungsmelder und photoelektrische Sensoren Bewegungen bzw. Licht im OP registriert bzw. nicht mehr registriert werden. Dennoch ist hierbei ein häufiger, aufwändiger Wechsel von Videobändern erforderlich. Nachteilig ist auch, dass die Speicherung aktiviert wird unabhängig von der Art der Aktivität im OP, also beispielsweise auch bei Reinigungs- oder Wartungsarbeiten oder auch beim Durchgang von Personen durch einen OP. Hierdurch werden Aufnahmen erzeugt, die für Archivierung bzw. Qualitätskontrolle nicht relevant sind, unnötig Speicherplatz verbrauchen und bei der Ermittlung und Auswertung relevanter Aufnahme unnötigen Aufwand erzeugen.

Es ist die Aufgabe der vorliegenden Erfindung, ein Verfahren und eine Vorrichtung anzugeben, womit eine fortlaufende Speicherung von medizinischen Daten, insbesondere Videodaten und/oder Sprachdaten aus einem OP, möglich ist, wobei die Speicherung nicht relevanter Daten weitgehend vermieden wird.

Diese Aufgabe wird durch ein Verfahren gemäß Anspruch 1, eine Vorrichtung gemäß Anspruch 6 gelöst.

Erfindungsgemäß werden die zu dokumentierenden Daten von mindestens einer Datenerfassungseinrichtung erfasst. Die Datenerfassungseinrichtung ist dabei insbesondere in einem Operationssaal angeordnet und kann beispielsweise eine Videokamera sein, die den gesamten oder einen Teil des Operationssaals aufnimmt. Die Datenerfassungseinrichtung kann aber auch Daten anderer Art aus dem OP aufnehmen, etwa Schall bzw. Sprache, oder Daten, die im OP befindliche chirurgische oder andere Geräte betreffen bzw. von diesen erzeugt werden. Die erfassten Daten können insbesondere in Echtzeit in eine Speichereinrichtung übernommen und auf einem Speichermedium gespeichert werden. Um die Speicherung nicht relevanter Daten und damit unnötigen Speicherungs- und Auswertungsaufwand zu vermeiden, erfolgt die Speicherung in Abhängigkeit von der Anwesenheit eines Patienten im Operationssaal. Insbesondere wird die Speicherung aktiviert, wenn der zu operierende Patient in den Operationssaal hineingebracht wird. Die Präsenz des Patienten im OP wird dabei durch Sensoren erkannt, die für mit dem Patienten verbundene Merkmale empfindlich sind, wodurch die Aufzeichnung automatisch gestartet bzw. wieder beendet wird.

In bevorzugter Weise wird die Speicherung deaktiviert, wenn der Patient den OP verlässt. Dabei kann direkt die Anwesenheit des Patienten im OP erkannt werden oder der Eintritt des Patienten in den OP bzw. das Verlassen des OPs durch den Patienten in einem Ein- bzw. Ausgangsbereich des OPs.

Gemäß einer bevorzugten Ausprägung des erfindungsgemäßen Verfahrens wird die Speicherung nur bei Präsenz einer weiteren vorbestimmten Person im Operationssaal aktiviert, die zusätzlich zum Patitienten im OP anwesend ist. Die weitere Person kann dabei insbesondere ein Arzt bzw. ein Anästhesist oder ein Chirurg sein, insbesondere derjenige, der für die betreffende Operation vorgesehen bzw. verantwortlich ist. Die Anwesenheit der weiteren Person kann insbesondere auf die gleiche Weise erkannt werden wie die des Patienten. Beginn und/oder Ende der Datenaufzeichnung können auch von der gleichzeitigen Präsenz von mehreren weiteren vorbestimmten Personen abhängig sein, beispielsweise von der gleichzeitigen Anwesenheit von Chirurg und Anästhesist. Ebenso kann die Speicherung von der Präsenz einer oder mehrerer Personen aus einer vordefinierten Gruppe abhängig sein, wenn beispielsweise eine Vertretung oder Ablösung des operierenden Chirurgen möglich sein soll.

Auf diese Weise wird die Speicherung nur dann aktiviert, wenn der chirurgische Eingriff stattfindet, und nicht während einer eventuellen Vorbereitungs- oder Wartezeit. Hierdurch kann weiterhin unnötiger Speicherungs- bzw. Auswertungsaufwand vermieden werden, wobei diejenigen Daten, die für die Qualitätssicherung, für die Dokumentation oder auch zum Nachvollziehen der Operation bei späterem Auftreten von Komplikationen notwendig sind, gespeichert werden.

Gemäß einer weiteren Ausprägung des erfindungsgemäßen Verfahrens werden die Daten zumindest teilweise aus einem Netzwerk, von einem Computersystem und/oder von einem medizinischen Gerät, insbesondere einer endoskopischen Videokamera, übernommen. Ein solches Netzwerk kann ein im OP installiertes busgestütztes Netzwerk zur Steuerung aller operationsrelevanten Funktionen sein, das auch mit einem übergeordneten Netzwerk, beispielsweise einem Krankenhaus-Informations-System (Hospital Information System, HIS) und/oder einem OP-Management- oder Planungssystem verbunden sein kann. Über ein solches Netzwerk können auch beispielsweise Patientendaten übernommen und gespeichert werden. Auch die Daten über die Kriterien für Beginn und/oder Ende der Speicherung, wie etwa die Identifikation des Patienten bzw. der weiteren Personen, können beispielsweise über ein solches Netzwerk übernommen werden.

Hierdurch ist auf einfache Weise die Verfügbarkeit aller relevanten Daten gesichert. Darüber hinaus kann die Speicherung an einem vom OP getrennten Ort stattfinden.

In bevorzugter Weise enthalten die Daten Bildinformationen, insbesondere endoskopische Bildinformationen. Solche Bildinformationen werden bei videogestützten endoskopischen Operationen erzeugt und stehen damit automatisch zur Speicherung zur Verfügung. Diese Bildinformationen sind darüber hinaus besonders relevant für die Qualitätssicherung, Dokumentation und ggf. für die nachträgliche Ermittlung von Ursachen möglicher Komplikationen. Ebenso können auch von einer Raumkamera aufgenommene Bilddaten und/oder Sprachdaten, beispielsweise über ein Mikrofon im OP aufgenommene Sprache und Geräusche oder von einer Sprachsteuerung erfasste Kommandos, gespeichert werden. Hierdurch ist eine Speicherung aller möglicherweise relevanten Daten gesichert.

In besonders bevorzugter Weise umfassen die Daten Informationen über Art und/oder Ort und/oder Funktion und/oder Parameter von chirurgischen Instrumenten und/oder Geräten. Solche chirurgischen Instrumente bzw. Geräte sind beispielsweise endoskopische Instrumente, Endoskopoptiken, Insufflatoren, HF-Generatoren usw. Für Dokumentationszwecke kann es wichtig sein, den Typ der verwendeten Instrumente bzw. Geräte und beispielsweise auch die Seriennummer zu speichern. Häufig verfügen die im OP verwendeten Geräte auch über eine Funktionskontrolle, die Daten beispielsweise über Druck und Fluss eines Insufflationsgases liefert. Sofern die Geräte in ein im OP installiertes Netzwerk integriert sind, stehen Daten über Art, Funktion und eingestellte Parameter der Geräte automatisch zur Speicherung zur Verfügung; auch weitere Geräte, wie etwas Raumbeleuchtung oder ein automatischer OP-Tisch, können hierin einbezogen sein. Auch der Ort der Instrumente bzw. Geräte im OP kann durch ein automatisches, ggf. drahtloses, System erfasst werden. Es kann aber auch vorgesehen sein, manuell eingegebene Daten über Art, Ort, Funktion und/oder Parameter der verwendeten Instrumente bzw. Geräte zu speichern.

Hierdurch ist eine besonders vollständige Speicherung aller möglicherweise relevanten Daten gewährleistet.

Erfindungsgemäß umfassen die gespeicherten Daten Anästhesiedaten, Narkoseüberwachungsdaten und/oder Vitalparameter des Patienten. Diese können beispielsweise von den betreffenden Geräten automatisch erfasst und über ein OP-Netzwerk zur Speicherung zur Verfügung gestellt werden. Diese Daten sind besonders wichtig zu dokumentieren, beispielsweise zur Auswertung bei möglichen späteren Komplikationen.

Erfindungsgemäß wird die Speicherung in Abhängigkeit von den Anästhesiedaten, Narkoseüberwachungsdaten und/oder Vitalparametern des Patienten aktiviert. Diese Daten zeigen u. a. an, wann die Narkose einsetzt bzw. beendet ist. Wenn beispielsweise die Speicherung von der Narkose abhängig ist, kann auf diese Weise erreicht werden, dass die Speicherung dann einsetzt, wenn der Patient in Narkose ist, während eine Speicherung nicht notwendig ist, wenn der Patient bei Bewusstsein ist. Dies kann eine zusätzliche Bedingung für die Aktivierung der Speicherung sein, zusätzlich zur Anwesenheit im OP, oder eine unabhängige Bedingung, insbesondere wenn auch der Bereich der Ein- bzw. Ausleitung der Narkose überwacht wird, beispielsweise durch zusätzliche Raumkameras, und die betreffenden Daten gespeichert werden sollen. Hierdurch wird dem Bedürfnis des Patienten, dass in Narkose wenigsten eine indirekte oder nachträgliche visuelle Kontrolle der Vorgänge bei der Operation möglich ist, Rechnung getragen, während für Vorgänge, die der Patient bewusst wahrnehmen kann, keine Speicherung notwendig ist.

Gemäß einer weiteren Ausgestaltung des erfindungsgemäßen Verfahrens werden die Präsenz und/oder Identität des Patienten und/oder weiterer Personen durch ein drahtloses Identifikations- bzw. Ortungssystem festgestellt. Ein solches System kann beispielsweise ein RFID (Radio Frequency Identification Device) - System sein. Hierdurch ist sowohl eine Identifikation möglich mit Hilfe von RFID-Tags, die dem Patienten bzw. den weiteren Personen zugeordnet sind und insbesondere am oder im Körper getragen werden. Zudem ist es möglich, drahtlos Informationen über den Ort der betreffenden RFID-Tags zu gewinnen, beispielsweise durch das Vorbeigehen an einem RFID-Lesegerät. Zusätzlich zur drahtlosen Identifikation kann auch die Identifikation über ein drahtgebundenes System erfolgen.

Erfindungsgemäß erfolgt die Speicherung in einem Ringspeicher, wobei für einen Benutzer ein Zugriff auf die Daten möglich ist und ein automatisches Löschen und/oder Überschreiben der Daten nach einer vorbestimmten Zeit erfolgt. Der Ringspeicher kann dabei in Hardware oder in Software, beispielsweise auf einem Server, ausgebildet sein.

Ein Löschen bzw. Überschreiben nach einer vorbestimmten Zeit hat den Vorteil, dass eine Zeitspanne angegeben werden kann, die sich nach den für die Dokumentation vorgegebenen Zeiträumen oder nach dem Zeitraum richtet, nach dem bei bestimmten Operationen üblicherweise keine Komplikationen mehr auftreten.

Eine erfindungsgemäße Vorrichtung umfasst eine Datenübernahmeeinrichtung zur Übernahme von Daten von mindestens einer Datenquelle. Die Datenquelle kann dabei eine Kamera sein, beispielsweise eine Raumkamera oder eine endoskopische Videokamera, oder auch andere Datenerfassungseinrichtungen, wie beispielsweise ein Mikrofon zur Aufnahme von Schalldaten, sowie die Datenübernahme von drahtlos lesbaren Datenträgern, wie beispielsweise RFID-Systemen, oder auch ein medizinisches Gerät oder eine Überwachungseinrichtung. Diese Daten können über eine Datenübernahmeeinrichtung, beispielsweise über eine Schnittstelle, die auch in das betreffende Gerät integriert sein kann, oder ein Netzwerk, das drahtgebundene oder drahtlose Übertragung ermöglichen kann, übernommen werden. Die Datenübernahmeeinrichtung kann zur Übernahme von Daten beispielsweise auch von einem Datenträger, wie einer CD oder DVD, ausgebildet sein.

Weiterhin umfasst die erfindungsgemäße Vorrichtung mindestens einer Speichereinrichtung zur Speicherung von Daten. Dies kann insbesondere ein Computer mit einem entsprechenden Speichermedium, beispielsweise einer Festplatte, einer DVD oder einem anderen geeigneten Speichermedium sein. Ferner sind mindestens eine Anzeigeeinrichtung zur Anzeige von Daten für einen Benutzer und mindestens einer Eingabeeinrichtung zur Eingabe von Daten und/oder Anweisungen durch den Benutzer vorgesehen. Die Anzeigeeinrichtung kann insbesondere ein Computerbildschirm sein oder andere Datenausgabeeinrichtungen wie etwa Lautsprecher. Zur Eingabe von Daten und Anweisungen kann beispielsweise eine Tastatur, ein Touch Screen, oder eine Sprachsteuerung zur Verfügung stehen.

Dadurch, dass eine Speichereinrichtung vorgesehen ist, die einen ersten Datenspeicher enthält, der als Ringspeicher ausgebildet ist, auf dem die dort gespeicherten Daten jeweils nach einer vorbestimmten Zeit, nachdem sie eingespeichert wurden, wieder gelöscht werden, ist sichergestellt, dass das Speichermedium nicht erschöpft wird, sondern stets zur Aufnahme neuer Daten zur Verfügung steht. Dabei kann das Löschen auch durch Überschreiben der Daten geschehen. Es kann auch vorgesehen sein, dass der Ringspeicher für ein Überschreiben der Daten ausgebildet ist, wenn ein vorbestimmter Speicherplatz erschöpft ist.

Ist aufgrund beispielsweise einer nach einer Operation aufgetretenen Komplikation ein Zugriff auf die bei dieser Operation aufgenommenen Daten notwendig, so kann der jeweils relevante Teil der Daten dem Ringspeicher entnommen und in einem anderen Speicher zur weiteren Verwertung gespeichert werden oder auf dem Ringspeicher wird keine weitere Datenspeicherung und damit auch keine weitere Datenlöschung vorgenommen, so dass die Daten zur Auswertung zur Verfügung stehen. Es kann daher auch ein weiterer Ringspeicher vorgesehen sein, der dann für die fortlaufende Datenspeicherung zur Verfügung steht.

Gemäß einer bevorzugten Ausführungsform enthält die Speichereinrichtung einen zweiten Datenspeicher, der nicht als Ringspeicher ausgebildet ist, auf dem ebenfalls Daten bevorzugt automatisch gespeichert werden. Dies hat den Vorteil, dass einige Daten, beispielsweise solche, die nur wenig Speicherplatz erfordern oder die langfristig gespeichert werden sollen, nicht von der regelmäßigen Löschung auf dem Ringspeicher betroffen sind.

Gemäß einer weiteren Ausführungsform kann der Benutzer die Übernahme von Daten in den ersten oder in den zweiten Datenspeicher steuern, d. h., der Benutzer kann angeben, welche Daten langfristig gespeichert werden sollen und welche nach einer vorbestimmten Zeit wieder gelöscht werden können. Dies kann, ebenso wie die Festlegung der vorbestimmten Zeit, für jeden Benutzer unterschiedlich geschehen, wobei jeweils eine Identifikation oder Autorisierung des Benutzers erforderlich gemacht werden kann. Damit ist je nach Art der Daten, beispielsweise für unterschiedliche Arten von Operationen, eine jeweils angepasste Festlegung und damit eine optimale Ausnutzung des Speicherplatzes möglich.

Gemäß einer weiteren bevorzugten Ausführungsform erfolgt die Übernahme von Daten in den ersten oder in den zweiten Datenspeicher zumindest teilweise aufgrund von vorbestimmten Kriterien, die nicht vom Benutzer beeinflussbar sind. Auch die Zeit, nach der die im Ringspeicher gespeicherten Daten automatisch gelöscht werden, kann benutzerunabhängig vorgegeben sein. Hierdurch ist die Erfüllung von Qualitätssicherungs- oder gesetzlichen Erfordernissen sicherer zu gewährleisten. Eine geeignete Zeitspanne, nach der die gespeicherten Daten, wenn hierauf kein Zugriff erfolgt ist, gelöscht werden können, ist beispielsweise ca. 4 Wochen, da sich in der Regel nach dieser Zeit etwaige Komplikationen nach einer Operation herausgestellt hätten. Je nach Art der Operation kann aber auch etwa eine Woche ausreichen oder eine Speicherung über einen Zeitraum von mehreren Monaten oder etwa einem Jahr notwendig sein.

Gemäß einer besonders bevorzugten Ausführungsform enthalten die gespeicherten Daten Bildinformationen, insbesondere endoskopische Bildinformationen, beispielsweise endoskopische Videobilder. Ebenso können die Videobilder einer Raumkamera, die zumindest den Bereich um den OP-Tisch herum erfasst, gespeichert werden. Dies hat den Vorteil, dass durch die Speicherung von Bilddaten eine besonders lückenlose Dokumentation der Vorgänge bei einer Operation möglich ist, beispielsweise die nachträgliche Erkennung von Fehlern oder Besonderheiten, die dem Operateur während der Operation entgangen waren. Ebenso können auch Sprachdaten erfasst werden, beispielsweise in eine Sprachsteuerung eingegebene Anweisungen, aber auch andere akustische Daten, wie etwa Gespräche oder Geräusche, aus einem OP. Auch dies trägt zu einer besonders vollständigen Dokumentation der Vorgänge bei einer OP bei.

Die erfindungsgemäße Vorrichtung kann insbesondere auch zur Bearbeitung der gespeicherten Daten, insbesondere der Bilddaten, ausgebildet sein. Der Benutzer hat somit die Möglichkeit, Kommentare, Dokumente, Rechercheergebnisse usw. zu den Bilddaten hinzuzufügen, Markierungen in den Bilddaten anzubringen usw.

In einer weiteren besonders bevorzugten Ausführungsform ist die Vorrichtung zur Aufnahme und Speicherung von Daten über die bei einer chirurgischen Operation verwendeten Instrumente und/oder Geräte ausgebildet. Diese Daten können beispielsweise Typ und Serien- oder Inventarnummer der betreffenden Instrumente oder Geräte enthalten. Hiermit ist eine vollständigere Dokumentation aller Vorgänge bei der Operation möglich. Ebenso können gleichzeitig Daten des Patienten erfasst werden, insbesondere Anamnesedaten, aber auch beispielsweise Vitalparameter während der Operation.

Gemäß einer weiteren bevorzugten Ausführungsform enthalten die Daten insbesondere Informationen über Ort und/oder Funktion und/oder Parameter der bei der chirurgischen Operation verwendeten Instrumente und/oder Geräte. Die Daten zur Funktion können insbesondere die eingestellten Parameter sein, wie beispielsweise Insufflationsdruck oder HF-Spannung, aber auch damit zusammenhängende Messwerte, wie beispielsweise gemessener Druck, Leckstrom, Temperatur usw. Darüber hinaus kann die Miterfassung von Anästhesiedaten und Vitalparametern möglich sein. So ist eine besonders vollständige Dokumentation der Vorgänge bei einer Operation möglich.

Gemäß einer weiteren besonders bevorzugten Ausführungsform werden die Daten automatisch übernommen. Dies kann sowohl die Übernahme der Bilddaten, wie auch die Übernahme der Instrumenten- und Gerätedaten betreffen. Hierdurch ist sichergestellt, dass unabhängig von der Situation im Operationssaal eine vollständige Dokumentation erfolgt.

Dabei können die Daten insbesondere zumindest teilweise aus einem Netzwerk, von einem Computersystem und/oder von medizinischen Geräten, insbesondere einer endoskopischen Videokamera, aber auch beispielsweise von einer Raumkamera übernommen werden. Insbesondere dann, wenn die erfindungsgemäße Vorrichtung zur Integration in ein busgesteuertes Kommunikationssystem innerhalb des Operationssaals ausgebildet ist, ist dies eine einfache und sichere Lösung, bei der auch alle weiteren operationellen und sicherheitsbedingten Anforderungen innerhalb eines Operationssaals erfüllt werden können. Beispielsweise ist die Einbettung sicherheitsrelevanter Funktionen in ein geschlossenes System möglich, während nicht sicherheitsrelevante Funktionen in einem offenen System ausgeführt werden können.

Gemäß einer weiteren bevorzugten Ausführungsform werden die Daten zumindest teilweise über eine drahtlose Verbindung übernommen. Derartige drahtlose Verbindungen sind in vielen Bereichen gängig, beispielsweise können über ein WLAN Daten innerhalb und außerhalb des OP übertragen werden. Dies hat den Vorteil, dass die Bewegung hindernde Kabelverbindungen entfallen.

Gemäß einer weiteren besonders bevorzugten Ausführungsform umfasst die Vorrichtung weiterhin mindestens eine RFID-Identifikations- und/oder Ortungsvorrichtung. Damit kann ohne weitere Eingaben des Benutzers automatisch festgestellt werden, welche, mit einem entsprechenden Codeträger versehenen Instrumente, Siebe, Geräte usw. bei einer Operation verwendet wurden, ggf. auch, in welcher Konfiguration und zu welcher Zeit.

Erfindungsgemäß wird die Speicherung von Daten im Ringspeicher durch das Vorhandensein eines Patienten im Operationssaal ausgelöst. Damit kann verhindert werden, dass nicht relevante Daten, wie Videobilder oder Sprachdaten aus einem nicht benutzten OP, unnötig Speicherplatz verbrauchen. Andererseits wird zuverlässig sichergestellt, dass dann, wenn die Daten relevant sein könnten, diese, beispielsweise zur Qualitätssicherung, auch gespeichert werden.

Erfindungsgemäß wird das Vorhandensein des Patienten durch ein RFID-System detektiert. Vorteilhafterweise kann auch gleichzeitig eine Identifikation des Patienten oder auch die Übertragung von patientenspezifischen Daten erfolgen. Damit können zur Dokumentation, aber auch intraoperativ dem Operateur sofort die Daten des Patienten zur Verfiigung stehen. Hierfür kann insbesondere der Patient oder das Transportmittel des Patienten beispielsweise einen RFID-Codeträger oder ein anderes Identifikationsmittel tragen.

Gemäß einer weiteren bevorzugten Ausführungsform wird dementsprechend die Speicherung von Daten im Ringspeicher automatisch beendet, wenn der Patient den OP verlässt. Auch hierdurch wird sichergestellt, dass nicht unnötig Speicherplatz belegt wird.

Ein erfindungsgemäßes System zur Durchführung medizinischer Eingriffe umfasst eine Einrichtung zur Steuerung und/oder Überwachung mindestens eines Geräts, mindestens ein Gerät zur Verwendung bei einem medizinischen Eingriff, das mit der Steuerungs- und/oder Überwachungseinrichtung verbunden ist, und eine Vorrichtung zur Dokumentation medizinischer Daten nach einem der vorstehenden Ansprüche. Ein solches Gesamtsystem unterstützt in einfacher und sicherer Weise die Ausführung und die automatische Dokumentation aller Vorgänge bei einer Operation.

Hierbei können die für eine vollständige Dokumentation notwendigen Daten insbesondere dann aufgenommen und gespeichert werden, wenn das System weiterhin Mittel zur Identifikation und/oder Ortsbestimmung von medizinischen Instrumenten, medizinischen Geräten und/oder Personen umfasst. Solche Mittel können insbesondere ein RFID-System sein.

Zur Erkennung, ob ein Patient in den OP hineingebracht wird oder den OP verlässt, sind im Eingangsbereich des OP in Bewegungsrichtung hintereinander mehrere Schleusen mit jeweils einem RFID-Lesegerät zum Beispiel an der Eingangstür des OP installiert. Ebenso kann auf diese Weise die Bewegungsrichtung von mit RFID-Codeträgern ausgestatteten Instrumenten, Sieben, Geräten, Personal usw. erkannt werden. Hierdurch kann unabhängig von der Detektierung innerhalb des OP festgestellt werden, welche Personen und/oder Gegenstände sich im OP befinden bzw. zu einer bestimmten Zeit befunden haben.

Für den Fall, dass beim Durchgang durch die Schleusen nur in einer der beiden Schleusen eine korrekte Detektierung erfolgt, kann es vorgesehen sein, dass durch ein zusätzliches Eingabemittel die Bewegungsrichtung automatisch ermittelt oder manuell eingegeben wird. So kann beispielsweise ein Warnsignal, etwa ein Warnton, ausgegeben werden. Die betreffende Person, deren Bewegungsrichtung nicht detektiert worden ist, kann dann beispielsweise über einen in der Nähe der Schleuse angeordneten Eingabeknopf die Bewegungsrichtung eingeben. Das gleiche gilt, wenn die Bewegungsrichtung des Patienten nicht eindeutig erkannt worden ist. Dies hat den Vorteil, dass zur Erkennung der Anwesenheit im OP kein erneuter Durchgang durch beide Schleusen erforderlich ist.

Weiterhin kann ein Personenidentifikationssystem vorgesehen sein, durch das die weiteren im OP anwesenden Personen eindeutig identifiziert werden können. Eine sichere Identifikation kann dabei insbesondere durch biometrische Kenndaten erfolgen. Die erfolgte Identifikation wird bevorzugt ebenfalls gespeichert. In besonders vorteilhafter Weise geschieht die Identifikation durch berührungslose Eingabesysteme, beispielsweise Iris-Biometrie oder Gesichtsvermessung mit nachfolgender automatischer Bildverarbeitung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung.

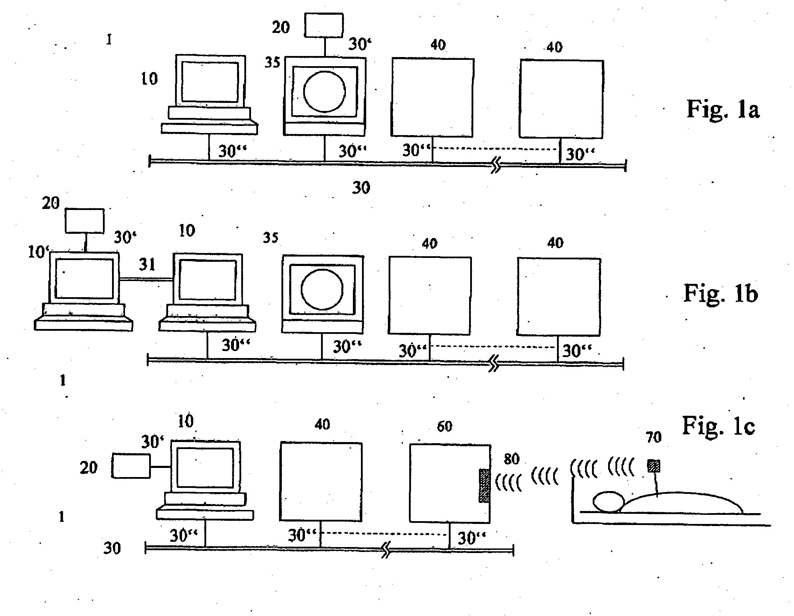

Figur 1 zeigt in schematischer Form bevorzugte Ausführungsbeispiele einer erfindungsgemäßen Vorrichtung.

Gemäß Fig. 1a dient ein Steuerrechner 10 als Eingabevorrichtung zur Eingabe von Anweisungen durch den Benutzer und zur Anzeige von aufgenommenen oder gespeicherten Daten, insbesondere Bilddaten. Der Steuerrechner 10 ist über einen Netzwerkanschluss 30" mit einem Netzwerk 30 verbunden. Über das Netzwerk 30 können Daten ausgetauscht werden. Durch einen weiteren Netzwerkanschluss 30" ist eine endoskopische Kamera 35, die zur Erfassung der mit einem Endoskop gewonnenen Bilddaten dient, mit dem Netzwerk 30 verbunden. Die endoskopische Kamera 35 ist durch die Verbindungsleitung 30', die den Austausch von Daten ermöglicht, an einen Ringspeicher 20 angeschlossen. An das Netzwerk 30 können durch weitere Anschlüsse 30'' weitere medizinische Geräte 40 angeschlossen werden. Dies können Geräte in beliebiger Anzahl sein, wie etwa Pumpen, HF-Generatoren, Insufflationseinrichtungen, Absaugeinrichtungen, Beatmungsvorrichtungen usw. Diese können beispielweise über das Netzwerk 30 die Werte der Betriebsparameter übertragen. Darüber hinaus können die Geräte über das Netzwerk 30 beispielweise angesteuert oder mit Strom versorgt werden.

Fig. 1b zeigt eine zweite bevorzugte Ausführungsform der erfindungsgemäßen Vorrichtung. Hierbei ist der Ringspeicher 20 über eine Verbindungsleitung 30' mit einer Eingabevorrichtung 10' in Form eines Personalcomputers verbunden. Der Personalcomputer 10' ist über eine Datenleitung 31 mit der Steuereinrichtung 10 des Netzwerks 30 verbunden. Die beispielweise durch die Kamera 35 erfassten Daten werden durch das Netzwerk 30 zur Steuereinrichtung 10 übertragen, die ihrerseits eine Übertragung der Daten auf den Personalcomputer 10' durch die Datenleitung 31 ermöglicht. Neben dem Ringspeicher 20 verfügt die Dokumentationsvorrichtung 1 in Fig. 1b über weitere Speicherkapazitäten, die jeweils in den Steuereinheiten 10' und 10 angeordnet sind und beispielsweise nach den jeweiligen Erfordernissen verwendet werden können.

Gemäß Fig. 1c kann darüber hinaus eine Empfangsstation 60 vorgesehen sein, die über einen Netzwerkanschluss 30" mit dem Netzwerk 30 verbunden ist. Auf der Empfangstation 60 ist ein Sensor 80, beispielsweise ein Radiosignalempfänger, angeordnet. Der Sensor 80 empfängt die Signale, die ein Transponder beispielsweise in Form einer an einem chirurgischen Instrument oder an einem Patienten angeordnete RFID-Markierung 70 aussendet. Dieses Signal kann beispielweise dazu dienen, dass über das Netzwerk 30 die Speicherung der über das Netzwerk 30 übertragenen Daten im Ringspeicher 20 gestartet wird.

Der übrige Teil der erfindungsgemäßen Vorrichtung gemäß dem Ausführungsbeispiel nach Fig. 1c kann in besonders bevorzugter Weise auch wie in Fig. 1b ausgebildet sein.

Gemäß einem Ausführungsbeispiel der vorliegenden Erfindung aktiviert der Patient, der im OP normalerweise nicht bei vollem Bewusstsein ist, durch das Tragen eines Identifikationselements am oder im Körper drahtlos die Sicherungsüberwachung bzw. Datendokumentation im Operationssaal. Die Aktivierung erfolgt zum Beispiel über ein RFID-Schleusen-System am Eingang in den OP. Der Patient trägt dabei ein RFID-Identifikationselement am bzw. im Körper. Die Speicherung der Daten im Ringspeicher kann durch eine Verknüpfung des Signals des RFID-Identifikationselements mit dem Vitalparameter der Narkoseüberwachung erfolgen, d.h. ist der Patient im OP-Saal und unter Narkose, so wird die Speicherung aktiviert. Dies wird dem Patientenwunsch gerecht, dass der Patient, obwohl er nicht bei Bewusstsein ist, eine gewisse indirekte audiovisuelle Kontrolle aktivieren kann.

Die RFID-Identifikation und/oder Ortungsvorrichtung besteht aus einem oder mehreren Identifikationselementen, welche der Patient und/oder das Operationspersonal trägt, und RFID-Empfangseinheiten, die die Informationen bzw. die Identifikationselemente auf berührungslosem Weg sicher erfassen. Idealerweise besteht eine Art Schleuse aus mehreren RFID-Empfangseinheiten, die räumlich hintereinander installiert sind, so dass eindeutig entschieden werden kann, ob der Patient gerade in den OP kommt, d. h., eingeschleust wird oder diesen gerade verlässt. Kommt der Patient in den OP, wird die Sicherheitsüberwachung und die Datenspeicherung gestartet, verlässt der Patient den OP, so wird die Aufzeichnung in den Ringpuffer gestoppt.

Die Speichereinrichtung kann an ein Personenidentifikationssystem gekoppelt sein, mit dem sich vor Beginn der Operation alle Mitglieder des OP-Teams eindeutig über biometrische Kenndaten, die im System hinterlegt sind, im Bezug zum Patienten und dessen anstehende Operation identifizieren. Aufgrund der Sterilsituation geschieht dies auf berührungslosem Weg mit speziellen kameragestützten Eingabesystemen, beispielsweise Iris-Biometrie und/oder Gesichtsvermessung. Diese Identifikation des OP-Teams gegenüber dem aktuellen Patienten und der anstehenden Operation wird ebenfalls dokumentiert und gespeichert.

## Patentansprüche

1. Verfahren zur Dokumentation medizinischer Daten, bei dem von mindestens einer Datenerfassungseinrichtung erfasste Daten aus einem Operationssaal in einem Speichermedium gespeichert werden, wobei die Daten Bildinformationen und/oder Sprachdaten enthalten, **dadurch gekennzeichnet, dass** die Speicherung nur bei Präsenz eines zu operierenden Patienten im Operationssaal aktiviert wird und dass die Speicherung in einem Ringspeicher erfolgt, wobei für einen Benutzer ein Zugriff auf die Daten möglich ist und ein Löschen und/oder Überschreiben nach einer vorbestimmten Zeit erfolgt, dass die Daten Anästhesiedaten, Narkoseüberwachungsdaten und/oder Vitalparameter des Patienten enthalten und dass die Speicherung in Abhängigkeit von den Anästhesiedaten, Narkoseüberwachungsdaten und/oder Vitalparametern des Patienten aktiviert wird, wobei die Präsenz des Patienten durch ein RFID-System festgestellt wird und wobei in einem Eingangsbereich des Operationssaals hintereinander mehrere Schleusen mit jeweils einem RFID-Lesegerät installiert sind und die Bewegungsrichtung des mit einem RFID-Codeträger ausgestatteten Patienten erkannt wird.

2. Verfahren nach Anspruch 1, **dadurch gekennzeichnet, dass** die Speicherung nur bei Präsenz einer weiteren vorbestimmten Person im Operationssaal aktiviert wird.

3. Verfahren nach Anspruch 1 oder 2, **dadurch gekennzeichnet, dass** die Daten zumindest teilweise aus einem Netzwerk, von einem Computersystem und/oder von einem medizinischen Gerät übernommen werden.

4. Verfahren nach einem der vorhergehenden Ansprüche, **dadurch gekennzeichnet, dass** die Daten Informationen über Art, Ort, Funktion und/oder Parameter von chirurgischen Instrumenten und/oder Geräten umfassen.

5. Verfahren nach einem der vorhergehenden Ansprüche, **dadurch gekennzeichnet, dass** die Präsenz und/oder Identität des Patienten und/oder weiterer Personen durch ein drahtloses Identifikations- bzw. Ortungssystem festgestellt wird.

6. Vorrichtung zur Dokumentation medizinischer Daten, mit mindestens einer Datenerfassungseinrichtung zur Erfassung von Daten aus einem Operationssaal, mindestens einer Datenübernahmeeinrichtung zur Übernahme der von der mindestens einen Datenerfassungseinrichtungen erfassten Daten und mindestens einer Speichereinrichtung zur Speicherung der Daten, wobei die Daten Bildinformationen und/oder Sprachdaten enthalten, **dadurch gekennzeichnet, dass** die Speicherung nur bei Präsenz des zu operierenden Patienten im Operationssaal aktiviert wird und dass die Speicherung in einem Ringspeicher erfolgt, wobei für einen Benutzer ein Zugriff auf die Daten möglich ist und ein Löschen und/oder Überschreiben nach einer vorbestimmten Zeit erfolgt, dass die Daten Anästhesiedaten, Narkoseüberwachungsdaten und/oder Vitalparameter des Patienten enthalten und dass die Speicherung in Abhängigkeit von den Anästhesiedaten, Narkoseüberwachungsdaten und/oder Vitalparametern des Patienten aktiviert wird, wobei die Präsenz des Patienten durch ein RFID-System festgestellt wird und wobei in einem Eingangsbereich des Operationssaals hintereinander mehrere Schleusen mit jeweils einem RFID-Lesegerät installiert sind und die Bewegungsrichtung des mit einem RFID-Codeträger ausgestatteten Patienten erkannt wird.

7. Vorrichtung nach Anspruch 6, **dadurch gekennzeichnet, dass** die Vorrichtung weiterhin mindestens eine Anzeigeeinrichtung zur Anzeige von Daten für einen Benutzer und mindestens eine Eingabeeinrichtung zur Eingabe von Daten und/oder Anweisungen durch den Benutzer umfasst und dass die Speichereinrichtung einen ersten Datenspeicher enthält, der zum automatischen Löschen und/oder Überschreiben gespeicherter Daten nach einer vorbestimmten Zeit ausgebildet ist.

8. Vorrichtung nach Anspruch 7, **dadurch gekennzeichnet, dass** die Speichereinrichtung einen zweiten Datenspeicher enthält, der nicht zum automatischen Löschen und/oder Überschreiben gespeicherter Daten nach einer vorbestimmten Zeit ausgebildet ist.

9. Vorrichtung nach Anspruch 7 oder 8, **dadurch gekennzeichnet, dass** die Vorrichtung weiterhin mindestens eine RFID-Identifikations- und/oder Ortungsvorrichtung im Eingangsbereich des Operationssaals umfasst.

10. Vorrichtung nach Anspruch 9, **dadurch gekennzeichnet, dass** mindestens ein Mittel zur Identifikation und/oder Ortsbestimmung von medizinischen Instrumenten, medizinischen Geräten und/oder Personen vorgesehen ist.

11. Vorrichtung nach einem der Ansprüche 6 bis 10, **dadurch gekennzeichnet, dass** diese zur Durchführung des Verfahrens gemäß einem der Ansprüche 1 bis 5 ausgebildet ist.

12. Vorrichtung nach einem der Ansprüche 6 bis 11, weiterhin umfassend
- eine Einrichtung zur Steuerung und/oder Überwachung mindestens eines Geräts und
- mindestens ein mit der Steuerungs- und/oder Überwachungseinrichtung verbundenes Gerät zur Verwendung bei einem medizinischen Eingriff.

## Claims

1. Method for documenting medical data, in which data from an operating theatre, acquired by at least one data acquisition apparatus, are stored in a storage medium, wherein the data contain image information and/or speech data, **characterized in that** storage is only activated if a patient to be operated on is present in the operating theatre and **in that** storage takes place in a circular buffer, wherein a user can access the data and there is deletion and/or overwriting after a predetermined period of time, **in that** the data contain anaesthesia data, anaesthesia monitoring data and/or vital parameters of the patient and **in that** storage is activated in a manner dependent upon the anaesthesia data, anaesthesia monitoring data and/or vital parameters of the patient, wherein the presence of the patient is determined by an RFID system and wherein several gates, each with one RFID reader, are installed in succession in an entrance region of the operating theatre and the movement direction of the patient equipped with an RFID code carrier is identified.

2. Method according to Claim 1, **characterized in that** storage is only activated if a further predetermined person is present in the operating theatre.

3. Method according to Claim 1 or 2, **characterized in that** the data are at least in part taken over from a network, from a computer system and/or from an item of medical equipment.

4. Method according to one of the preceding claims, **characterized in that** the data comprise information in respect of type, location, function and/or parameters of surgical instruments and/or equipment.

5. Method according to one of the preceding claims, **characterized in that** the presence and/or identity of the patient and/or further persons is determined by a wireless identification or locating system.

6. Device for documenting medical data, with at least one data acquisition apparatus for acquiring data from an operating theatre, at least one data take-over apparatus for taking over the data acquired by the at least one data acquisition apparatus and at least one storage apparatus for storing the data, wherein the data contain image information and/or speech data, **characterized in that** storage is only activated if the patient to be operated on is present in the operating theatre and **in that** storage takes place in a circular buffer, wherein a user can access the data and there is deletion and/or overwriting after a predetermined period of time, **in that** the data contain anaesthesia data, anaesthesia monitoring data and/or vital parameters of the patient and **in that** storage is activated in a manner dependent upon the anaesthesia data, anaesthesia monitoring data and/or vital parameters of the patient, wherein the presence of the patient is determined by an RFID system and wherein several gates, each with one RFID reader, are installed in succession in an entrance region of the operating theatre and the movement direction of the patient equipped with an RFID code carrier is identified.

7. Device according to Claim 6, **characterized in that** the device furthermore comprises at least one display apparatus for displaying data to a user and at least one input apparatus for entering data and/or instructions by the user and **in that** the storage apparatus contains a first data storage medium, which is embodied for automatic deletion and/or overwriting of stored data after a predetermined period of time.

8. Device according to Claim 7, **characterized in that** the storage apparatus contains a second data storage medium, which is not embodied for automatic deletion and/or overwriting of stored data after a predetermined period of time.

9. Device according to Claim 7 or 8, **characterized in that** the device furthermore comprises at least one RFID identification device and/or locating device in the entrance region of the operating theatre.

10. Device according to Claim 9, **characterized in that** at least one means for identifying and/or determining the location of medical instruments, medical equipment and/or persons is provided.

11. Device according to one of Claims 6 to 10, **characterized in that** it is embodied for carrying out the method according to one of Claims 1 to 5.

12. Device according to one of Claims 6 to 11, furthermore comprising:
- an apparatus for controlling and/or monitoring at least one item of equipment and
- at least one item of equipment connected to the control apparatus and/or monitoring apparatus, for use during a medical intervention.

## Revendications

1. Procédé pour la documentation de données médicales, dans lequel des données acquises par au moins un dispositif d'acquisition de données et provenant d'une salle opération sont stockées sur un support de stockage, les données contenant des informations d'images et/ou des données vocales, **caractérisé en ce que** le stockage n'est activé qu'en présence d'un patient à opérer dans la salle d'opération et **en ce que** le stockage s'effectue dans un tampon circulaire, un accès aux données étant possible à un utilisateur et un effacement et/ou un écrasement étant effectué(s) après un temps prédéterminé, **en ce que** les données contiennent des données d'anesthésie, des données de surveillance de l'anesthésie et/ou des paramètres vitaux du patient et **en ce que** le stockage est activé en fonction des données d'anesthésie, des données de surveillance de l'anesthésie et/ou des paramètres vitaux du patient, la présence du patient étant déterminée au moyen d'un système RFID et une pluralité de portillons chacun équipés d'un lecteur RFID respectif étant installés les uns derrière les autres dans une zone d'entrée de la salle d'opération et la direction de déplacement du patient équipé d'un badge RFID étant détectée.

2. Procédé selon la revendication 1, **caractérisé en ce que** le stockage n'est activé qu'en présence d'une autre personne prédéterminée dans la salle d'opération.

3. Procédé selon la revendication 1 ou 2, **caractérisé en ce que** les données proviennent au moins en partie d'un réseau, d'un système informatique et/ou d'un appareil médical.

4. Procédé selon l'une quelconque des revendications précédentes, **caractérisé en ce que** les données comprennent des informations concernant le type, l'emplacement, la fonction et/ou des paramètres d'instruments et/ou d'appareils chirurgicaux.

5. Procédé selon l'une quelconque des revendications précédentes, **caractérisé en ce que** la présence et/ou l'identité du patient et/ou d'une autre personnes est déterminée au moyen d'un système d'identification ou de positionnement sans fil.

6. Dispositif de documentation de données médicales, comportant au moins un dispositif d'acquisition de données destiné à acquérir des données provenant d'une salle opération, au moins un dispositif de réception de données destiné à recevoir les données acquises par l'au moins un dispositif d'acquisition de données et au moins un dispositif de stockage destiné à stocker les données, les données contenant des informations d'images et/ou des données vocales, **caractérisé en ce que** le stockage n'est activé qu'en présence du patient à opérer dans la salle d'opération, et **en ce que** le stockage s'effectue dans un tampon circulaire, un accès aux données étant possible à un utilisateur et un effacement et/ou un écrasement étant effectué(s) après un temps prédéterminé, **en ce que** les données contiennent des données d'anesthésie, des données de surveillance de l'anesthésie et/ou des paramètres vitaux du patient et **en ce que** le stockage est activé en fonction des données d'anesthésie, des données de surveillance de l'anesthésie et/ou des paramètres vitaux du patient, la présence du patient étant déterminée au moyen d'un système RFID et une pluralité de portillons chacun équipés d'un lecteur RFID respectif étant installés les uns derrière les autres dans une zone d'entrée de la salle d'opération et la direction de déplacement du patient équipé d'un badge RFID étant détectée.

7. Dispositif selon la revendication 6, **caractérisé en ce que** le dispositif comprend en outre au moins un dispositif d'affichage destiné à afficher des données pour un utilisateur et au moins un dispositif de saisie permettant à l'utilisateur de saisir des données et/ou des instructions et **en ce que** le dispositif de stockage contient une première mémoire de données qui est conçue pour effacer et/ou écraser automatiquement des données stockées après un temps prédéterminé.

8. Dispositif selon la revendication 7, **caractérisé en ce que** le dispositif de stockage contient une seconde mémoire de données qui est conçue pour ne pas effacer et/ou écraser automatiquement des données stockées après un temps prédéterminé.

9. Dispositif selon la revendication 7 ou 8, **caractérisé en ce que** le dispositif comprend en outre au moins un dispositif d'identification et/ou de positionnement RFID dans une zone d'entrée de la salle d'opération.

10. Dispositif selon la revendication 9, **caractérisé en ce qu'**il est prévu au moins un moyen destiné à identifier et/ou déterminer la position d'instruments médicaux, d'appareils médicaux et/ou de personnes.

11. Dispositif selon l'une quelconque des revendications 6 à 10, **caractérisé en ce qu'**il est conçu pour mettre en oeuvre le procédé selon l'une quelconque des revendications 1 à 5.

12. Dispositif selon l'une quelconque des revendications 6 à 11, comprenant en outre
- un moyen de commande et/ou de surveillance d'au moins un appareil, et
- au moins un appareil connecté au moyen de commande et/ou de surveillance et destiné à être utilisé lors d'une intervention médicale.
